# EUROPÄISCHE PATENTSCHRIFT

(11) **EP 0 591 297 B1**
(45) Veröffentlichungstag und Bekanntmachung des Hinweises auf die Patenterteilung: **23.04.1997**
(21) Anmeldenummer: 92912732.2
(22) Anmeldetag: 17.06.1992
(51) Int. Cl.: C07C 45/48, C07C 49/04, C07C 49/203

(54) **VERFAHREN ZUR HERSTELLUNG VON FETTKETONEN**
METHOD OF PREPARING KETONES DERIVED FROM FATTY ACIDS
PROCEDE DE PREPARATION DE CETONES DERIVEES D'ACIDES GRAS

(30) Priorität: 26.06.1991 DE 4121117
(43) Veröffentlichungstag der Anmeldung: 13.04.1994
(73) Patentinhaber: Henkel Kommanditgesellschaft auf Aktien, 40191 Düsseldorf (DE)
(72) Erfinder: WESTFECHTEL, Alfred, D-4010 Hilden (DE); BREUCKER, Christoph, D-5657 Haan 2 (DE); GUTSCHE, Bernhard, D-4010 Hilden (DE); JEROMIN, Lutz, D-4010 Hilden (DE); EIERDANZ, Horst, D-4010 Hilden (DE); BAUMANN, Horst, D-5653 Leichlingen (DE); SCHMID, Karl-Heinz, D-4020 Mettmann (DE); NONNENKAMP, Werner, D-5460 Linz (DE)
(86) Internationale Anmeldenummer: EP9201373
(87) Internationale Veröffentlichungsnummer: WO9300320

(56) Entgegenhaltungen:
- US-A- 3 391 191
- JOURNAL OF THE SOCIETY OF CHEMICAL INDUSTRY, Bd. 66, 1947, London, GB, Seite 402-407; R.G. CURTIS et al: "The ketonization of higher fatty acids with some observations on the mechanism of the reaction. Part I. Studies of waxes"

## Beschreibung

Die Erfindung betrifft ein Verfahren zur Herstellung von Fettketonen durch Pyrolyse von Fettsäure-Magnesiumsalzen.

### Stand der Technik

Langkettige symmetrische und asymmetrische Ketone auf Basis von Fettsäuren, sogenannte "Fettketone", werden entweder direkt oder nach Sulfonierung oder Quaternierung als Zusätze zu Weichspülern, Haarwaschmitteln, Korrosionsinhibitoren oder Flotationshilfsmitteln eingesetzt **[Chem.Ing.-Tech. 62, 416 u. 512 (1990)]**.

Die Herstellung von Fettketonen durch Pyrolyse von Fettsäuresalzen in Gegenwart von katalytischen Mengen Calcium-, Eisen oder Mangansalzen ist seit dem Jahre 1855 bekannt und durch ein umfangreiches Schrifttum belegt **[Houben-Weyl, Methoden der Organischen Chemie, Thieme-Verlag, Stuttgart, Bd. 7/2a, S.622 ff]**.

In neuer Zeit hat sich die Verwendung von Magnesiumoxid als Katalysator bewährt. So wird beispielsweise in **Org.Synth., Vol.33, S.854ff** ein Laborverfahren zur Herstellung von Stearon beschrieben, bei dem man 1 Mol Stearinsäure und 0,5 Mol Magnesiumoxid bei 340°C zur Reaktion bringt. Zur Aufarbeitung ist es erforderlich, das Rohprodukt zunächst mit Schwefelsäure, Wasser und Natriumhydrogencarbonat-Lösung zu waschen, wobei das reine Stearon erst nach zweimaligem Kristallisieren erhalten wird. Ferner wird empfohlen, bei der Reaktion auftretenden Schaum entweder mechanisch zu zerstören oder durch Antischaummittel zu bekämpfen. Zusammengenommen kommt ein derartiges Verfahren infolge des mit der Aufarbeitung verbundenen hohen apparativen Aufwands für eine technische Nutzung nicht in Betracht.

Auch aus **J.Soc.Chem.Ind. 66, 402 (1947)** ist ein Verfahren zur Herstellung von Fettketonen durch Pyrolyse von Magnesiumsalzen von Fettsäuren bekannt. Zur Bewältigung der Schaumprobleme wird hierin vorgeschlagen, die Fettsäure im Bezug auf das Magnesiumoxid zunächst im deutlichen Unterschuß vorzulegen, die Reaktionsmischung aufzuheizen und die restliche Fettsäure dann kontinuierlich zuzudosieren. Auf diesem Wege können die Schaumprobleme zwar minimiert, jedoch nicht gänzlich abgestellt werden, so daß nach wie vor ein Teil der Fettsäure ausgetragen wird und ohne zusätzlichen Maßnahmen somit verloren geht. Ein weiterer Nachteil des Verfahrens besteht darin, daß das Magnesiumsalz nach der Reaktion durch Filtration, Sedimentation oder Behandlung mit Mineralsäuren abgetrennt werden muß und somit nicht quantitativ aus den Produkten entfernt werden kann.

Die Aufgabe der Erfindung bestand somit darin, ein neues Verfahren für die Herstellung von Fettketonen zu entwickeln, das frei von den geschilderten Nachteilen ist.

### Beschreibung der Erfindung

Gegenstand der Erfindung ist ein Verfahren zur Herstellung von Fettketonen durch Pyrolyse von Magnesiumsalzen von Fettsäuren der Formel (I)

R¹-COOH (I)

in der R¹CO für einen gesättigten aliphatischen Acylrest mit 12 bis 22 Kohlenstoffatomen steht, bei dem man Magnesiumoxid zunächst mit einem Teil der Fettsäure in Kontakt bringt, die Mischung auf eine Reaktionstemperatur im Bereich von 320 bis 345°C aufheizt und anschließend die restliche Menge Fettsäure nachdosiert, das sich dadurch auszeichnet, daß man
(a) Fettsäure und Magnesiumoxid im molaren Verhältnis 1 : 0,5 bis 1 : 1 umsetzt,
(b) die gebildeten Fettketone unter vermindertem Druck von 1 bis 50 mbar aus der Reaktionsmischung abdestilliert und
(c) den Destillationssumpf unter Zugabe einer der verbrauchten Fettsäure entsprechenden Menge wieder in die Reaktion zurückführt.

Obschon die Herstellung von Fettketonen durch Pyrolyse von Fettsäure-Magnesiumsalzen schon seit langem bekannt ist, wurde erst jetzt überraschenderweise gefunden, daß sich die Schaumentwicklung und die damit verbundenen Probleme bei der Herstellung unterbinden lassen, wenn man die Fettketone bei der Reaktionstemperatur unter vermindertem Druck aus der Reaktionsmischung abdestilliert. Auf diesem Wege werden Verluste an Ausgangsmaterial verhindert und Produkte erhalten, die gegenüber dem Stand der Technik nur noch einen minimalen Magnesiumgehalt aufweisen. Durch den Umstand, daß die Fettketone durch die Destillation nur eine kurze Verweilzeit in der heißen Reaktionszone besitzen und dadurch eine vergleichsweise geringe thermische Belastung erfahren, werden überraschend hell farbige Produkte erhalten; die Farbträger verbleiben im Destillationssumpf. Desweiteren schließt die Erfindung die Erkenntnis ein, daß das bei der Reaktion anfallende Sumpfprodukt unmittelbar wieder mit Fettsäure umgesetzt werden kann, so daß eine halbkontinuierliche Durchführung des Verfahrens möglich ist. Weitere Vorteile des Verfahrens sind in der isothermen Durchführung des Verfahrens und den kurzen Chargenzeiten zu sehen. Eine vollständige Pyrolyse ist nicht erforderlich, da die als Zwischenprodukte gebildeten Magnesiumseifen, die nicht pyrolysiert werden, im Sumpf verbleiben.

**Fettsäuren**, die im Sinne der Erfindung als Ausgangsstoffe in Betracht kommen, sind Laurinsäure, Myristinsäure, Palmitinsäure, Stearinsäure, Arachinsäure oder Behensäure. Bevorzugt ist der Einsatz von Fettsäuren der Formel (I), in der R¹CO für einen Acylrest mit 16 bis 18 Kohlenstoffatomen, insbesondere für einen Stearylrest steht.

Wie in der Fettchemie üblich, können die Fettsäuren auch in Form technischer Schnitte eingesetzt werden, wie sie z. B. durch Druckspaltung von Fetten und Ölen, beispielsweise Palmöl, Palmkernöl, Kokosöl, oder Rindertalg erhalten werden.

In einer bevorzugten Ausführungsform des Verfahrens bringt man das Magnesiumoxid zunächst mit 5 bis 20 Gew.-% der Gesamtmenge an Fettsäure in Kontakt, heizt die Mischung auf die Reaktionstemperatur auf und dosiert anschließend die restliche Menge Fettsäure zu.

Die Gewinnung der reinen Produkte aus der Reaktionsmischung erfolgt ohne weitere Aufreinigung, indem man die bei der Pyrolyse gebildeten Fettketone bei der Reaktionstemperatur unter einem verminderten Druck von 1 bis 50 mbar abdestilliert.

### Gewerbliche Anwendbarkeit

Die erhaltenen Fettketone sind ohne Bleiche hellfarbig und weisen äußerst geringe Magnesiumgehalte auf. Sie eignen sich als Additive für Weichspüler, Haarwaschmittel, Korrosionsinhibitoren oder Flotationshilfsmittel, in denen sie in Mengen von 0,1 bis 25, vorzugsweise 1 bis 10 Gew.-% - bezogen auf die Mittel - enthalten sein können. Desweiteren können sie durch Sulfonierung oder Quaternierung in oberflächenaktive Substanzen überführt werden.

Die folgenden Beispiele sollen den Gegenstand der Erfindung näher erläutern, ohne ihn darauf einzuschränken.

### Beispiele

### Beispiel 1:

**Herstellung von Pentatriacontan-18-on (Stearon)**. In einem Dreihalskolben mit Tropftrichter und Destillationsbrücke wurde ein Gemisch aus
57 g (0,2 Mol) Octadecansäure und
81 g (2,0 Mol) Magnesiumoxid
auf 340°C erhitzt. Nach 60 min Reaktionszeit wurden weitere 1026 g (3,6 Mol) Octadecansäure zugetropft, wobei CO₂ und Wasser freigesetzt wurden. Die Reaktionsmischung wurde weitere 60 min bei der Temperatur gehalten und anschließend das Reaktionsprodukt bei vermindertem Druck (p = 10 mbar) vom Sumpf abdestilliert. Die Ausbeute an Stearon (Destillat) betrug 70 % der theoretischen Menge, der Mg-Gehalt < 50 ppm.

### Beispiel 2:

Das unter Vakuum befindliche Sumpfprodukt aus Beispiel 1 wurde weiterverarbeitet. Zunächst wurde das Vakuum durch Einleiten von Stickstoff gebrochen und dem Sumpf bei einer Temperatur von 340°C weitere 912 g (3,2 Mol) Stearinsäure zudosiert. Die weitere Verarbeitung der Reaktionsmischung erfolgte analog Beispiel 1; die Ausbeute an Stearon betrug abermals 70 % der theoretischen Menge.

Der Cyclus wurde 10mal wiederholt. Die Ausbeute pro Cyclus betrug konstant ca. 70 % der theoretischen Menge.

## Patentansprüche

1. Verfahren zur halbkontinuierlichen Herstellung von Fettketonen durch Pyrolyse von Fettsäure-Magnesiumsalzen, bei dem man
a) Fettsäuren der Formel (I),
**R**^{**1**}**COOH** (I)
in der R¹CO für einen gesättigten aliphatischen Acylrest mit 12 bis 22 Kohlenstoffatomen steht, und Magnesiumoxid im molaren Verhältnis 1 : 1 bis 1 : 2 bei Temperaturen im Bereich von 320 bis 345°C umsetzt, wobei man das Magnesiumoxid zunächst mit 5 bis 20 Gew.-% der Gesamtmenge an Fettsäure in Kontakt bringt, die Mischung auf die Reaktionstemperatur aufheizt und anschließend die restliche Menge Fettsäure nachdosiert,
b) die gebildeten Fettketone unter vermindertem Druck von 1 bis 50 mbar aus der Reaktionsmischung abdestilliert und
c) den Destillationssumpf unter Zugabe einer der verbrauchten Fettsäure entsprechenden Menge wieder in die Reaktion einsetzt.

2. Verfahren nach Anspruch 1, **dadurch gekennzeichnet**, daß man Fettsäuren der Formel (I) einsetzt, in der R¹CO für einen Acylrest mit 16 bis 18 Kohlenstoffatomen steht.

## Claims

1. A process for the semi-continuous production of fatty ketones by pyrolysis of fatty acid magnesium salts, in which
a) fatty acids corresponding to formula (I):
R¹COOH (I)
in which R¹CO is a saturated aliphatic acyl radical containing 12 to 22 carbon atoms,
and magnesium oxide are reacted in a molar ratio of 1:1 to 1:2 at temperatures of 320 to 345°C, the magnesium oxide initially being contacted with 5 to 20% by weight of the total quantity of fatty acid, the mixture being heated to the reaction temperature and the remaining fatty acid being subsequently added,
b) the fatty ketones formed are distilled off from the reaction mixture under a reduced pressure of 1 to 50 mbar and
c) the bottom distillation product is reintroduced into the reaction in a quantity corresponding to the fatty acid consumed.

2. A process as claimed in claim 1, characterized in that fatty acids corresponding to formula (I), in which R¹CO is an acyl radical containing 16 to 18 carbon atoms, are used.

## Revendications

1. Procédé de préparation semi-continue de cétones grasses par pyrolyse de sels de magnésium d'acides gras, dans lequel
a) on mélange des acides gras de la formule (I),
R¹COOH (I)
dans laquelle R¹CO représente un radical acyle aliphatique saturé comportant 12 à 22 atomes de carbone, et de l'oxyde de magnésium, dans un rapport molaire de 1:1 à 1:2, à des températures comprises dans l'intervalle de 320 à 345 °C, dans lequel on met l'oxyde de magnésium tout d'abord en contact avec 5 à 20 % en poids de la quantité totale de l'acide gras, chauffe le mélange à la température réactionnelle, et on ajoute ensuite la quantité restante d'acide gras,
b) on chasse les cétones grasses du mélange réactionnel, par distillation, sous pression réduite de 1 à 50 mbars et
(c) on recycle dans la réaction le fond de distillation, en ajoutant une quantité correspondante à l'acide gras consommé.

2. Procédé selon la revendication 1, caractérisé en ce que l'on utilise des acides gras de la formule (I), dans laquelle R¹CO représente un radical acyle comportant 16 à 18 atomes de carbone.
